# NEUE EUROPÄISCHE PATENTSCHRIFT

(11) **EP 2 116 249 B2**
(45) Veröffentlichungstag und Bekanntmachung des Hinweises auf die Entscheidung über den Einspruch: **05.04.2023**
(45) Hinweis auf die Patenterteilung: 02.05.2012
(21) Anmeldenummer: 09075120.7
(22) Anmeldetag: 15.05.2006
(51) Int. Cl.: A61K 31/519, A61K 31/135, A61K 31/7028, A61K 31/00, A61K 31/565, A61K 31/57, A61K 31/525, A61K 31/585, A61K 31/4415, A61P 15/18, A61P 15/12, A61K 31/56, A61K 45/06

(54) **Pharmazeutische Zusammensetzung enthaltend Gestagene und/oder Estrogene und 5-Methyl-(6S)-tetrahydrofolat**
Pharmaceutical compound containing gestagens and/or oestrogens and 5-methyl-(6S)-tetrahydrofolates
Composition pharmaceutique contenant un gestagène et/ou un estrogène et de la 5-méthyle-(6S)-tétrahydrofolate

(30) Priorität: 13.05.2005 DE 102005023301; 03.04.2006 DE 102006016285
(43) Veröffentlichungstag der Anmeldung: 11.11.2009
(62) Teilanmeldung aus: 06753787.8
(73) Patentinhaber: Bayer Intellectual Property GmbH, 40789 Monheim (DE); Merck & Cie, 6460 Altdorf (CH)
(72) Erfinder: Strothmann, Kai, 61440 Oberusel (DE); Smith, Gavin, 13505 Berlin (DE); King, Kristina, 10437 Berlin (DE); Moser, Rudolf, 8200 Schaffhausen (CH); Pietrzik, Klaus, 53340 Meckenheim (DE)
(74) Vertreter: BIP Patents

(56) Entgegenhaltungen:
- EP-A2- 0 898 965
- WO-A-03/070255

## Beschreibung

Die vorliegende Erfindung betrifft ein Verfahren zur Formulierung einer pharmazeutischen Zusammensetzung, die Gestagene, Estrogene und 5-Methyl-(6S)-tetrahydrofolat enthält, wobei die pharmazeutische Formulierung als orales Kontrazeptivum eingesetzt werden kann und dabei folatmangelbedingten Erkrankungen und Fehlbildungen vorbeugt, ohne dabei die Symptome eines Vitamin-B₁₂-Mangels zu maskieren.

Die auf dem Gebiet der Fertilitätskontrolle tätigen Pharmaunternehmen sind stetig bemüht, die vorhandenen Kontrazeptiva zu verbessern. Dazu gehört nicht nur die Erhöhung der kontrazeptiven Sicherheit durch Entwicklung neuer Substanzen und ein verbesserter Anwendungskomfort. Vielmehr werden auch innovative Ansätze zur Kombination von Kontrazeption und Krankheitsvorbeugung verfolgt.

Eine Anzahl von Erkrankungen wird als mit einem Folatmangel in Zusammenhang stehend angesehen. So kann die Verabreichung von Folaten zum Beispiel in Form von Folsäure das Risiko von Herz-/Kreislauferkrankungen sowie bestimmter maligner Erkrankungen (wie zum Beispiel Brust- und Colonkarzinom) minimieren.

Entwicklungsstörungen von Ungeborenen sind besonders schwerwiegende Folgen eines Folatmangels bei Frauen im gebärfähigen Alter. So haben Frauen mit niedrigen Folatspiegeln gegenüber solchen mit ausreichend hohen Folatspiegeln ein erhöhtes Risiko, Kinder zu gebären, die an angeborenen Fehlbildungen wie Neuralrohr-, Ventrikelklappen- und Urogenitaldefekten leiden.

Neuralrohrdefekte sind die häufigsten angeborenen Fehlbildungen des Zentralnervensystems. Sie entstehen durch einen unvollständigen Verschluss des Neuralrohrs etwa in der dritten bis vierten Woche der embryonalen Entwicklung. Zu den Neuralrohrdefekten gehören die Spina bifida (teilweise mit Meningozele oder Meningomyelozele), die Enzephalozele bzw. Anenzephalien, die durch das teilweise oder vollständige Fehlen von Hirnarealen gekennzeichnet sind. Kinder mit Anenzephalie sind praktisch nicht überlebensfähig.

Die Spina bifida zeichnet sich durch einen unvollständigen Wirbelbogenschluss aus. Sie führt je nach Art der Läsion zu lebenslangen Behinderungen in Form von unterschiedlichen sensiblen, aber auch motorischen Ausfällen so sind zum Beispiel zwei Drittel der Kinder und Erwachsenen durch Muskellähmungen rollstuhlabhängig. Eine Therapie erfolgt durch Deckung des Defekts, der Legung eines Shunts zum Ableiten des Liquors und langwierige orthopädische und neurologische Rehabilitation. Die für die medizinische Behandlung aufzuwendenden Kosten liegen bei durchschnittlich 500.000 € pro Kind.

Jährlich wird von circa 250.000 Neugeborenen mit Neuralrohrdefekten weltweit ausgegangen. In Deutschland und den USA liegt die Rate bei etwa 1 - 2 geschädigten Neugeborenen je 1.000 Geburten. In Deutschland werden jährlich ca. 500 Säuglinge mit Neuralrohrdefekten lebend geboren, bei weiteren 500 Schwangerschaften erfolgt ein Schwangerschaftsabbruch aufgrund pränataler Ultraschalldiagnose.

Ausreichend hohe Folatspiegel zum Zeitpunkt der Empfängnis und in der ersten Schwangerschaftsphase sind entscheidend zur Vermeidung von Neuralrohrdefekten. Allgemein wird ein Erythrozytenfolatspiegel von mindestens 906 nmol/l als erstrebenwert zur Reduktion der Häufigkeit von Neuralrohrdefekten angesehen.

Es ist bekannt, dass die rechtzeitige perikonzeptionelle Einnahme von Folsäure Neuralrohrdefekte um 50 - 70 % reduzieren kann. In den USA hat die dort praktizierte Anreicherung von Lebensmitteln mit Folsäure die Inzidenz von Neuralrohrdefekten bereits deutlich verringert; in Canada und Chile sogar um mehr als 50 %.

Sowohl eine freiwillige Anreicherung von Lebensmitteln wie zum Beispiel in Deutschland als auch die Einnahme von Folsäure-Präparaten erreicht jedoch nicht alle gebärfähigen Frauen in ausreichendem Maße. Zum einen sind sich viele Frauen der Gefahr von Neuralrohrdefekten und der Möglichkeit, ein entsprechendes Risiko durch Einnahme von Folsäure zu minimieren, nicht bewusst. So nehmen in vielen Ländern weit weniger als 10 % von ihnen perikonzeptional Folsäure-Präparate ein. Zum anderen sind trotz moderner und immer einfacher anzuwendender Verhütungsmethoden eine hohe Anzahl von Schwangerschaften - in den USA schätzungsweise bis zu 50 % (Inst. of Medicine 1998, NEJM 2004) - ungeplant, so dass eine bewusste Einnahme von Folsäurepräparaten vor der Empfängnis von vornherein ebenfalls ausscheidet. Darüber hinaus nehmen zum Beispiel in den USA circa 5 - 8 % der Anwenderinnen orale Kontrazeptiva nicht zuverlässig ein.

Dem Patent US 6,190,693 (Kafrissen et al.) lag daher die Aufgabe zugrunde, bestimmte mittels Folsäure behandelbare Erkrankungen von Konsumentinnen oraler Kontrazeptiva vorzubeugen. Kafrissen löste diese Aufgabe durch Zugabe von Folsäure zu einem oralen Kontrazeptivum. Er offenbarte eine Methode zur Folsäureverabreichung unter Verwendung einer pharmazeutischen Zusammensetzung, die sowohl gängige kontrazeptiv wirkende Substanzen als auch Folsäure enthielt.

Das Einfügen von Folsäure in orale Kontrazeptiva birgt jedoch ein ernstes Gesundheitsrisiko in sich, da dies die frühen, noch behandelbaren Symptome eines Vitamin-B₁₂-Mangels, wie zum Beispiel eine megaloblastische Anämie maskieren kann. Die durch einen Vitamin-B₁₂-Mangel hervorgerufenen hämatologischen Symptome lassen sich durch eine zusätzlich Folatgabe nämlich so gut behandeln, dass ein Vitamin-B₁₂-Mangel gar nicht, beziehungsweise nur noch sehr schwer erkennbar ist und daher in der Konsequenz nicht diagnostiziert wird. Die neuropsychiatrischen Symptome, wie zum Beispiel Paresthesie und Ataxie bleiben dann aber unbehandelt und könnten sich irreversibel verschlechtern.

Der Patentanmeldung WO 03/070255 (Coelingh Bennink) lag daher die Aufgabe zugrunde, ein Gesundheitsrisiko, welches bei Konsumentinnen Folsäure enthaltender oraler Kontrazeptiva durch die Maskierung der Symptome eines Vitamin-B₁₂-Mangels entsteht, zu vermeiden. Coelingh Bennink löst diese Aufgabe durch die Zugabe von Vitamin B₁₂ zu einem oralen Kontrazeptivum. Er offenbart ein Kit zur oralen, hormonellen Kontrazeption welches Estrogene und/oder Gestagene, Tetrahydrofolate und zwingend Vitamin B₁₂ enthält.

Ein weiteres Problem bei der Verabreichung von Folsäure- und Tetrahydrofolatpräparaten - die kein 5-Methyl-(6S)-tetrahydrofolat enthalten - ist der bei ca. 55 % der kaukasischen Bevölkerung mischerbig und bei circa 10 - 15 % reinerbig vorkommende Polymorphismus der Methylentetrahydrofolatreduktase (MTHFR C677T). Dieser Polymorphismus führt zu einer reduzierten Aktivität der Methylentetrahydrofolatreduktase, so dass die betroffenen Frauen das angebotene Folat und Tetrahydrofolat nicht in ausreichendem Maße in das im Organismus aktive 5-Methyl-(6S)-tetrahydrofolat metabolisieren können. Dieser Polymorphismus ist ein anerkannter Risikofaktor folatmangelbedingter Erkrankungen, insbesondere für Neuralrohrdefekte.

Als weiteres Problem kommt erschwerend hinzu, dass Folsäure kein natürlicherweise in Nahrungsmitteln vorkommender Stoff ist. Um biologisch wirksam zu sein, muss er im Stoffwechsel zuerst durch das Enzym Dihydrofolatreduktase in 7,8-Dihydrofolat und (6S)-Tetrahydrofolat umgewandelt werden. Die metabolische Kapazität, insbesondere der erste Aktivierungsschritt, zur Umwandlung des Provitamins Folsäure in seine aktiven reduzierten Formen ist limitiert und variiert zudem stark von Individuum zu Individuum. Da das Enzym Dihydrofolatreduktase beim Metabolismus von Metafolin keine Rolle spielt, sind Interaktionen zwischen Arzneimitteln, die die Dihydrofolatreduktase inhibieren, wie z. B. Methotrexat und der Dihydrofolatreduktase nicht zu erwarten.

Um auch Frauen, die unter Methylentetrahydrofolat-Reduktase-Defizienz leiden, ausreichend mit Folat zu versorgen, schlägt EP 0898965 (Müller et al.) die Verwendung von 5-Methyl-(6S)-tetrahydrofolsäure beziehungsweise entsprechender pharmazeutisch verträglicher Salze als Nahrungsmittelergänzung oder als Bestandteil von Arzneimitteln vor. EP 1044975 A1 offenbart unter anderem stabile kristalline Salze der 5-Methyl-(6S)-tetrahydrofolsäure und Verfahren zu deren Herstellung.

Die US 2006/034954 A1 offenbart eine pharmazeutische Formulierung enthaltend 5-Methyltetrahydrofolat, Phytoestrogene und Vitamin B6.

Es ist bekannt, dass ein großer Teil der Schwangerschaften kurz nach dem Absetzen des Kontrazeptivums eintritt (Farrow et al., Human Reproduction Vol. 17, No, 10, S. 2754 - 2761, 2002). Bei unregelmäßiger und unzuverlässiger Applikation kann es sogar während der Einnahme zur Schwangerschaft kommen. Ebenfalls ist bekannt, dass ein Mensch auch nach Beendigung einer zusätzlichen Folatapplikation noch für weitere etwa 90 Tage hiervon profitieren kann (FDA Advisory Committee for Reproductive Health Drugs (ACRHD): The public health issues, including the safety and potential clinical benefit, associated with combining folic acid and an oral contraceptive into a single combination product. 15. Dezember 2003; Summary Minutes, Frage 4). Voraussetzung dafür ist jedoch, dass in einem ausreichend langen vorangegangenen Zeitraum Folsäure in genügend hohem Maße zusätzlich zur normalen Nahrung aufgenommen wurde. Dieser sogenannte Gewebedepoteffekt ist durch erhöhte Folatspiegel in den Erythrozyten sichtbar.

Weiterhin ist bekannt, dass niedrige Folat-/hohe Homocysteinspiegel mit mehrfachen Spontanaborten assoziiert sind (Merlen et al., Obstet. et Gynecol. 2000, 95: S. 519 - 524).

Der vorliegenden Erfindung liegt die Aufgabe zu Grunde, ein Verfahren zur Formulierung eines Arzneimittels zu zu schaffen, welches zwar folatmangelbedingte Krankheiten vorzubeugen in der Lage ist, dabei aber, die Symptome eines Vitamin-B₁₂-Mangels nicht zu maskieren vermag.

Die Aufgabe wird erfindungsgemäß durch ein Verfahren zur Formulierung einer pharmazeutischen Zusammensetzung enthaltend ein oder mehrere Gestagene und optional Vitamin B₆ und/oder Vitamin B₂, Estrogene und 0,4 bis 1 mg des Kalziumsalzes der 5-Methyl-(6S)-tetrahydrofosäure, sowie pharmazeutisch verträgliche Hilfs- und Trägerstoffe in der Abwesenheit von Vitamin B12 gelöst, wobei das Kalziumsalz der 5-Methyl-(6S)-tetrahydrofolsäure erst nach dem Granulieren aufgezogen wird.

Der Erfindung liegt die, gegenüber WO03/070255 überraschende Erkenntnis zu Grunde, dass das Aufziehen von 5-Methyl-(6S)-tetrahydrofolat nach dem Granulieren erfolgen kann. Eine Gabe von Vitamin B₁₂ ist nicht erforderlich, um das, in WO 03/070255 geschilderte Gesundheitsrisiko zu vermeiden. Trotz der Verabreichung von 5-Methyl-(6S)-tetrahydrofolat kann ein Arzt einen Vitamin-B₁₂-Mangel diagnostizieren und gegebenenfalls behandeln.

Im Falle eines bestehenden Vitamin-B₁₂-Mangels kann Vitamin B₁₂ dann natürlich zusätzlich verabreicht werden. Der Zusatz weiterer Vitamine, wie zum Beispiel Vitamin B₆ oder Vitamin B₂ ist ebenfalls optional möglich. Überraschend gegenüber WO 03/070255 ist auch die Erkenntnis, dass ungleich der Gabe von Folaten oder anderen Tetrahydrofolaten einzig die Verwendung von 5-Methyl-(6S)-tetrahydrofolat in einem Kontrazeptivum auch im Falle eines homo- bzw. heterozygot vorliegenden Polymorphismus der Methylentetrahydrofolatreduktase die uneingeschränkte und ausreichende Verwendbarkeit der Folatkomponente durch den Organismus und damit deren biologische Aktivität zur Vermeidung von angeborenenfolatmangelbedingten Fehlbildungen möglich macht.

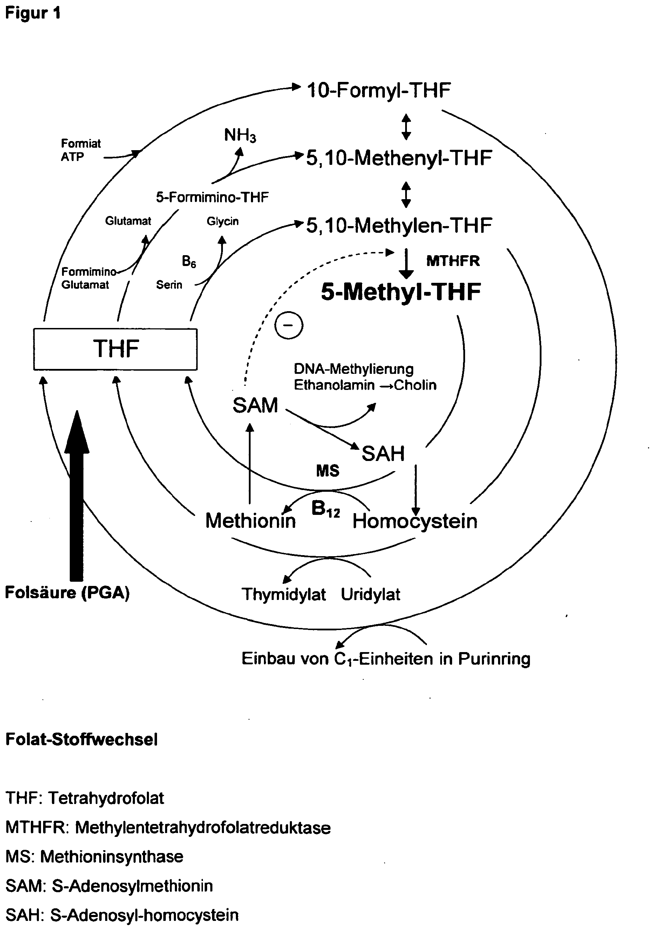

5-Methyl-(6S)-tetrahydrofolat wird im Stoffwechsel (siehe Figur 1) aus 5,10-Methylen-(6R)-tetrahydrofolat synthetisiert. Diese biochemische Reaktion wird durch das Enzym Methylentetrahydrofolatreduktase (MTHFR), von dem verschiedene genetische Mutationen bekannt sind, die sich zum Teil in einer eingeschränkten biologischen Aktivität manifestieren (MTHFR C677T Polymorphismus), katalysiert. 5-Methyl-(6S)-tetrahydrofolat wird in einem weiteren Schritt, welcher durch das Enzym Methioninsynthase (MS) katalysiert wird, in Tetrahydrofolat umgewandelt. Dabei erfolgt die Übertragung der 5-Methylgruppe von 5-Methyl-(6S)-tetrahydrofolat auf die Aminosäure Homocystein (Hcy), die dabei in die Aminosäure Methionin (Met) umgewandelt wird. Diese Vitamin-B₁₂-abhängige Reaktion wird im Homocysteinstoffwechsel auch als Homocysteinremethylierung bezeichnet. 5-Methyl-(6S)-tetrahydrofolat nimmt in der Gruppe der reduzierten Folate eine besondere Stellung ein, da 5-Methyl-(6S)-tetrahydrofolat nur durch die Homocysteinremethylierungsreaktion in Tetrahydrofolat umgewandelt werden kann. Tetrahydrofolat ist das eigentliche Trägermolekül für Einkohlenstoffeinheiten unterschiedlicher Oxidationsstufen. Im Stoffwechsel kann 5-Methyl-(6S)-tetrahydrofolat nur aus 5,10-Methylen-(6R)-tetrahydrofolat synthetisiert und nur durch Umwandlung in Tetrahydrofolat weiter metabolisiert werden. Die erste enzymatische Reaktion (MTHFR) ist unter physiologischen Bedingungen nicht reversibel, die zweite enzymatische Reaktion (MS) ist Vitamin-B₁₂ abhängig, das heißt bei Vitamin-B₁₂-Mangel akkumuliert 5-Methyl-(6S)-tetrahydrofolat und kann nicht weiter verstoffwechselt werden. Dieses Phänomen ist auch unter dem Begriff Methylfalle (methyl trap) bekannt. Nur 5-Methyl-(6S)-tetrahydrofolat, nicht aber alle übrigen oxidierten und reduzierten Folate wie Folsäure, 7,8-Dihydrofolat, (6S)-Tetrahydrofolat, 5-Formyl-(6S)-tetrahydrofolat, 10-Formyl-(6R)-tetrahydrofolat, 5,10-Methenyl-(6R)-tetrahydrofolat, 5,1 0-Methylen-(6R)-tetrahydrofolat, 5-Formimino-(6S)-tetrahydrofolat, weist diese besondere Eigenschaft auf. 5-Methyl-(6S)-tetrahydrofolat ist das einzige natürlich vorkommende Folat, welches einen Vitamin-B₁₂-Mangel nicht maskiert. Dies ist von besonderer Bedeutung bei Verwendung von 5-Methyl-(6S)-tetrahydrofolat in Kombination mit oralen Kontrazeptiva.

Als Gestagene können in der erfindungsgemäß hergestellten pharmazeutischen Zusammensetzung die folgenden Sub-stanzen Verwendung finden: Levonorgestrel, Norgestimat, Norethisteron, Dydrogesteron, Drospirenon, 3-beta-Hydro-xydesogestrel, 3-Ketodesogestrel (= Etonogestrel), 17-Deacetylnorgestimat, 19-Norprogesteron, Acetoxypregnenolon, Allylestrenol, Amgeston, Chlormadinon, Cyproteron, Demegeston, Desogestrel, Dienogest, Dihydrogesteron, Dimethi-steron, Ethisteron, Ethynodioldiacetat, Flurogestonacetat, Gastrinon, Gestoden, Gestrinon, Hydroxymethylprogesteron, Hydroxyprogesteron, Lynestrenol (= Lynoestrenol), Mecirogeston, Medroxyprogesteron, Megestrol, Melengestrol, No-megestrol, Norethindron (= Norethisteron), Norethynodrel, Norgestrel (einschließlich d-Norgestrel und dl-Norgestrel), Norgestrienon, Normethisteron, Progesteron, Quingestanol, (17alpha)-17-Hydroxy-11-methylen-19-norpregna-4,15-dien-20-yn-3-on, Tibolon, Trimegeston, Algeston acetophenid, Nestoron, Promegeston, 17-Hydroxyprogesteronester, 19-Nor-17hydroxyprogesteron, 17alpha-Ethinyl-testosteron, 17alpha-ethinyl-19-nor-testosteron, d-17beta-Acetoxy-13beta-ethyl-17alpha-ethinyl-gon-4-en-3-onoxim Tanaproget. Bevorzugt sind Levonorgestrel, Norgestimat, Norethiste-ron, Drospirenon, Dydrogesteron. Besonders bevorzugt ist Drospirenon.

Als Estrogene kommen Ethinylestradiol, Mestranol, Quinestranol, Estradiol, Estron, Estran, Estriol, Estetrol und konjugierte equine Estrogene in Frage. Bevorzugt sind dabei Ethinylestradiol, Estradiol und Mestranol, besonders bevorzugt ist Ethinylestradiol.

Die erfindungsgemäß verwendeten Mengen der jeweiligen Gestagene und/oder Estrogene entsprechen den in Kontrazeptiva üblicherweise bekannten Mengen.

Diese betragen normalerweise zum Beispiel für die nachfolgend genannten Gestagene:

| | |
|---|---|
| Drospirenon | 0,5 - 5 mg |
| Levonorgestrel | 30 - 250 µg |
| Norgestimat | 180 - 250 µg |
| Norethisteronacetat | 0,5 - 1 mg |
| Cyproteronacetat | 1 - 2 mg |
| Desogestrel | 20 - 150 µg |
| Dienogest | 2 - 3 mg |
| Gestoden | 60 - 75 µg |
| Tibolon | 2,5 mg |

Gemäß vorliegender Erfindung beträgt die täglich verabreichte bevorzugte Menge zum Beispiel an Drospirenon 0,5 bis 5 mg, besonders bevorzugt 3 mg.

Die erfindungsgemäß verwendete Estrogenmenge beträgt für die nachfolgend genannten Estrogene in etwa:

| | |
|---|---|
| Ethinylestradiol | 10 - 50 µg |
| Estradiol | 1 - 4 mg |
| Mestranol | 50 µg |

Gemäß der vorliegenden Erfindung beträgt die täglich verabreichte bevorzugte Menge zum Beispiel an Ethinylestradiol 10 bis 50 µg, besonders bevorzugt 10 bis 30 µg, ganz besonders bevorzugt 20 bis 30 µg.

Als 5-Methyl-(6S)-tetrahydrofolat wird das Kalziumsalz der 5-Methyl-(6S)-tetrahydrofolsäure (N-[4-[[(2-amino-1,4,5,6,7,8-hexahydro-4-oxo-5-methyl-(6S)-pteridinyl)methyl]amino]benzoyl]-L-glutaminsäu re) verwendet.

Die verwendete Menge zum Beispiel des Kalziumsalzes der 5-Methyl-(6S)-tetrahydrofolsäure(Metafolin) liegt zwischen 0,4 bis 1 mg, besonders bevor-zugt 451 µg (entsprechend 400 µg Folsäure bzw. 416 µg 5-Methyl-(6S)-tetrahydrofolsäure).

Als Modifikation des Kalziumsalzes der 5-Methyl-(6S)-tetrahydrofolsäure bevorzugt eingesetzt werden kristalline Modifikationen gemäss EP 1044975.

Optional kann Vitamin B₆ oder Vitamin B₂ enthalten sein. Zur Ausführung der Erfindung ist ein entsprechender Zusatz jedoch nicht erforderlich. Vitamin B₆ kann in einer Dosis zwischen 1 mg und 5 mg, vorzugsweise bei normal dosierter Anwendung zwischen 1 mg und 3 mg pro Tag verwendet werden. Vitamin B₂ kann in einer Dosis zwischen 1 mg und 5 mg, vorzugsweise bei normal dosierter Anwendung zwischen 1 mg und 2 mg pro Tag und bei hoch dosierter Anwendung zwischen 2 und 5 mg pro Tag eingesetzt werden.

Die Gestagene und/oder Estrogene sind dabei die kontrazeptiv wirksamen Substanzen. 5-Methyl-(6S)-tetrahydrofolat wird als Vitamin zugesetzt, um folatmangelbedingten Erkrankungen und Fehlbildungen vorzubeugen, ohne dabei jedoch die Symtome eines eventuell vorhandenen Vitamin-B₁₂-Mangels zu maskieren. Zusätzlich profitieren auch jene Frauen von 5-Methyl-(6S)-tetrahydrofolat, die aufgrund einer verminderten MTHFR Enzymaktivität (MTHFR C677T Polymorphismus) nur eingeschränkt zur Metabolisierung von Folsäure aber auch von reduzierten Folaten in der Lage sind.

In der bevorzugten Variante der vorliegenden Erfindung beträgt die täglich verabreichte Menge an Drospirenon 0,5 bis 5 mg, vorzugsweise 3 mg, die von Ethinylestradiol 10 bis 50 µg, vorzugsweise 10 bis 30 µg, besonders bevorzugt 20 bis 30 µg. Das Kalziumsalz der 5-Methyl-(6S)-tetrahydrofolsäure ist in dieser bevorzugten Variante der vorliegenden Erfindung in einer Menge von 0, 1 bis 10 mg, vorzugsweise 0,4 bis 1 mg, besonders bevorzugt 451 µg (entsprechend 400 µg Folsäure) enthalten.

Die Formulierung der pharmazeutischen Präparate auf Basis der neuen pharmazeutischen Zusammensetzung erfolgt in an sich bekannter Weise, indem man die Wirkstoffe mit den in der Galenik gebräuchlichen Trägersubstanzen, Füllstoffen, Zerfallsbeeinflussern, Bindemitteln, Feuchthaltemitteln, Gleitmitteln, Absorptionsmitteln, Verdünnungsmitteln, Geschmackskorrigentien, Färbemitteln und so weiter verarbeitet und in die gewünschten Applikationsformen, die auch Retardformen einschließen, überführt.

Im Arzneimittel können das Estrogen und das Gestagen sowie das 5-Methyl-(6S)-tetra-hydrofolat in gemeinsamen Dosierungseinheiten vorhanden sein. Das Estrogen mit dem Gestagen einerseits sowie das 5-Methyl-(6S)-tetrahydrofolat andererseits können aber auch in separaten Dosiseinheiten formuliert werden.

Sowohl Vitamin B₁₂, als auch 5-Methyl-(6S)-tetrahydrofolat sind gegenüber Luftsauerstoff und Luftfeuchte instabil. Bei dem Versuch Ethinylestradiol und Vitamin B₁₂ miteinander zu formulieren, wurde eine Inkompatibilität dieser beiden Substanzen untereinander festgestellt. Die Messungen der Inkompatibilitäten zwischen den vorgesehenen Formulierungsbestandteilen wurden mittels Thermoanalytischer Methode (DSC, Differential Scanning Calorimetry) durchgeführt. Inkompatibilitäten können hierbei durch geringere Schmelzenthalpien und Schmelztemperaturen erkannt werden. Diese werden zum Beispiel durch einen verminderten Anteil an kristalliner Substanz und den Anstieg von Verunreinigungen verursacht. In der Bestimmung wurden binäre Mischungen von Hilfs-oder Wirkstoffen jeweils mit Vitamin B₁₂ untersucht und die Kompatibilität unter dem Einfluss verschiedener Gase und Temperaturen überprüft. Vitamin B₁₂ zeigte bei den beschriebenen Untersuchungen eine starke Interaktion mit Ethinylestradiol. Die Ergebnisse der Inkompatibilitätsmessungen können der Tabelle 1 entnommen werden.

**Tabelle 1: Zusammenfassung der Kompatibilitätsuntersuchung**

| **Substanz** | **Kompatibilität** | **Art der Kompatibilität** | **Kommentare** |
|---|---|---|---|
| Drospirenon | + | vorwiegend gut | O₂ sensitiv |
| Ethinylestradiol | --- | starke Interaktion | stark 0₂ sensitiv |
| Ethinylestradiol β-Cyclodextrinkomplex | + | vorwiegend gut unter 60°C | 0₂ sensitiv, feuchtigkeitsempfindlich |
| Lactose | + | vorwiegend gut unter 60°C | 0₂ sensitiv, feuchtigkeitsempfindlich |
| Maisstärke | ++ | gut unter 60°C | 0₂ sensitiv, feuchtigkeitsempfindlich |
| Modifizierte Maisstärke | ++ | gut unter 60°C | 0₂ sensitiv, feuchtigkeitsempfindlich |
| Polyvinylpyrrolidon | + | vorwiegend gut unter 60°C | 0₂ sensitiv, feuchtigkeitsempfindlich |
| Magnesiumstearat | ++/- | indifferent, gut unter 60°C | 0₂ sensitiv, feuchtigkeitsempfindlich |
| Hydroxypropylmethylcellulose | ++ | gut unter 60°C | 0₂ sensitiv, feuchtigkeitsempfindlich |
| Hydroxypropylcellulose | ++ | gut unter 60°C | 0₂ sensitiv, feuchtigkeitsempfindlich |
| Maltodextrin | +/- - | indifferent, gut unter 60°C | 0₂ sensitiv, feuchtigkeitsempfindlich |
| Polyethylenglykol 6000 | - | Interaktion mit Feuchtigkeit | 0₂ sensitiv, feuchtigkeitsempfindlich |
| Lackmix | + | vorwiegend gut | 0₂ sensitiv, feuchtigkeits- |
| | | unter 60°C | empfindlich |
| Legende: | | | |
| ++ gute Kompatibilität erwartet unterhalb der angegebenen Temperatur | | | |
| + Kompatibilität unterhalb der angegebenen Temperatur | | | |
| ++/- indifferente Kompatibilität, möglicherweise gute Kompatibilität unterhalb der angegebenen Temperatur | | | |
| +/- - indifferente Kompatibilität, scheint unterhalb der angegebenen Temperatur kompatibel zu sein | | | |
| - (---) (starke) Interaktion, inkompatibel | | | |

Polyvinylpyrrolidon (PVP) ist wegen seiner benetzenden Eigenschaften für Hormonformulierungen besonders geeignet (Moneghini et al., Int J Pharm 175, 1998, 177 - 183). Eine Formulierung von 5-Methyl-(6S)-tetrahydrofolat mit PVP beschleunigt jedoch den Abbau des 5-Methyl-(6S)-tetrahydrofolat (vergleiche Tabelle 2 und 3; Prozess 3).

Eine weitere Aufgabe die dieser Anmeldung zugrunde lag und die mit der vorliegenden Erfindung gelöst wird, ist daher die Schaffung einer Möglichkeit Ethinylestradiol in Gegenwart von 5-Methyl-(6S)-tetrahydrofolat und optional von Vitamin B₁₂ stabil zu formulieren.

Es wurde herausgefunden, dass die Inkompatibilität zwischen Ethinylestradiol und Vitamin B₁₂ überraschenderweise dadurch verhindert werden kann, dass das Ethinylestradiol bei der Formulierung als Ethinylestradiol-beta-Cyclodextrin-Komplex (Ethinylestradiol als β-Cyclodextrin-Clathrat; Herstellung vergleiche WO02/49675) eingesetzt wird.

Entsprechende erfindungsgemäß hergestellte Formulierungen sind in Ausführungsbeispiel 1 (vergleiche Zusammenset-zung A, B und D) beschrieben.

Sie enthalten unter anderem eine Mischung aus Maisstärke und modifizierter Maisstärke (Starch). Stärke besteht aus Amylose und Amylopectin. Beides sind Polysaccharide basierend auf α-Glucoseeinheiten. Anstelle der Maisstärke können in pharmazeutischen Formulierungen aber auch zum Beispiel Reisstärke, Kartoffelstärke oder Weizenstärke verwendet werden. Die Stärke wird gequollen, suspendiert oder gelöst als Binderflüssigkeit oder als Feststoff eingesetzt. Sie kann unmodifiziert oder teilweise modifiziert sein. Die erfindungsgemäß bevorzugt verwendete Maisstärke hat die empirische Formel (C₆H₁₀O₅)ₙ mit n = 300 - 1000. lhr Molekulargewicht beträgt 50,000 - 160,000.

Die in pharmazeutischen Formulierungen verwendete Stärke dient nur zu einem Teil als reiner Füllstoff. Zu einem anderen Teil findet sie als Binder Verwendung. Erfindungsgemäß sind 1 - 5 %, bevorzugt -1,8 - 3 % des Tablettengewichts als Binder in Form von Maisstärke zuzusetzen. Neben der Maisstärke können als Binder auch Starch, eine Starchverbindung wie Maltodextrin, oder Zellulosederivate, wie zum Beispiel Carboxylmethylzellulose, Ethylzellulose, Hydroxypropylzellulose, Hydroxypropylmethylzellulose oder Methylzellulose eingesetzt werden. Erfindungsgemäß bevorzugt werden niedrig substituierte Zellulosederivate verwendet. Diese zeigen in einer 2 prozentigen wässrigen Lösung eine Viskosität von 1 - 20 mPas. Erfindungsgemäß bevorzugt sind Derivate mit einer Viskosität von 2 - 20 mPas, besonders bevorzugt solche mit einer Viskosität von 3 - 6 mPas.

In der erfindungsgemäß bevorzugt hergestellten Formulierung kann ein Teil der verwendeten Maisstärke durch niedrig substituierte Hydroxypropylzellulose (HPC) in einer Konzentration von 0,5 - 5 % (w/w), bevorzugt 1 - 3 % (w/w), besonders bevorzugt 2 % (w/w) ersetzt werden. Niedrig substituiert ist das Hydroxypropylzellulose vorliegend immer dann, wenn nicht weniger als 5 % und nicht mehr als 16 % seiner Hydroxylgruppen verestert oder verethert sind.

In Tabelle 2 ist der Gehalt an 5-Methyl-(6S)-tetrahydrofolat je Tablette in % bezogen auf den Sollgehalt von 100% in Abhängigkeit vom verwendeten Binder zeitlich unmittelbar nach der Herstellung dargestellt. Der dargestellte Gehalt an 5-Methyl-(6S)-tetrahydrofolat wurde im Content Uniformity Test (CUT) ermittelt. Die Herstellung der untersuchten Formulierung (Prozess 2) erfolgte durch Mischen der Bestandteile, Granulierung mit dem als Binder verwendetem Teil der Maisstärke, Aufziehen des 5-Methyl-(6S)-tetrahydrofolat nach Abschluss des Granulationsprozesses, erneutem Mischen und Tablettierung. Im Vergleich dazu wurde der Formulierung nach Prozess 3 Polyvinylpyrrolidon anstelle der Maisstärke als Binder zugesetzt. Der Gehalt an 5-Methyl-(6S)-tetrahydrofolat in der nach Prozess 3 hergestellten Formulierung ist geringer.

**Tabelle 2: Metafolingehalt in Abhängigkeit vom Binder direkt nach Herstellung**

| | Metafolingehalt Aufziehen, PVP (Prozess 3) | Metafolingehalt Aufziehen, Maisstärke (Prozess 2) |
|---|---|---|
| Mittelwert | 90,5 % | 96,1 % |

In Tabelle 3 ist der Gehalt an 5-Methyl-(6S)-tetrahydrofolat in Abhängigkeit vom verwendeten Binder nach einmonatiger Lagerung bei bestimmten Temperaturen und Luftfeuchtigkeiten dargestellt. Der in Tabelle 2 erkennbare Trend, dass mit PVP formuliertes 5-Methyl-(6S)-tetrahydrofolat instabiler ist, bestätigt sich insbesondere unter Lagerbedingungen von 40°C und 75 % relativer Luftfeuchtigkeit (rH).

**Tabelle 3: Metafolingehalt in Abhängigkeit vom Binder nach Lagerung**

| | 25°C 160% rH Aufziehen PVP (Prozess 3) | 25°C / 60% rH Aufziehen Maisstärke (Prozess 2) | 40°C / 75% rH Aufziehen PVP (Prozess 3) | 40°C / 75% rH Aufziehen Maisstärke (Prozess 2) |
|---|---|---|---|---|
| Vials offen | 89,5% | 92,1% | 37,7% | 67,7% |

Die Herstellung einer oralen Formulierung erfolgt normalerweise mittels Granulierung, Tablettierung und Befilmung. 5-Methyl-(6S)-tetrahydrofolat wird aber aufgrund seiner Sauerstoff- und Feuchtigkeitsempfindlichkeit bereits während der Granulation abgebaut. Besonders auffällig ist aber der weitere Abbau von 5-Methyl-(6S)-tetrahydrofolat bei der Lagerung. In einer Formulierung, in der - wie üblich - alle Komponenten des Arzneimittels einschließlich 5-Methyl-(6S)-tetrahydrofolat zuerst gemischt und erst danach granuliert werden, blieb nach einer Lagerzeit von einem Monat bei 40°C und 75 % relativer Luftfeuchtigkeit in geschlossenen Vials nur noch ein Rest von knapp 60 % (vergleiche Tabelle 5) des ursprünglich eingesetzten 5-Methyl-(6S)-tetrahydrofolat erhalten. Die Verluste während des Granulierungsprozesses können verringert werden, indem das 5-Methyl-(6S)-tetrahydrofolat erst nach dem Abschluss des Granulationsprozesses aufgezogen wird. Die trockene Zumischung während der Herstellung führt insoweit zu einer Stabilisierung des 5-Methyl-(6S)-tetrahydrofolat. Überraschenderweise bewirkt dies aber darüber hinaus auch noch eine weitere Stabilisierung während der Lagerung. Der Gehalt an 5-Methyl-(6S)-tetrahydrofolat, in einer durch späteres Aufziehen hergestellten Formulierung, liegt bei gleichen Lagerzeiten unter identischen Bedingungen oberhalb von 90 % (vergleiche Tabelle 5).

Tabelle 4 zeigt den Gehalt an 5-Methyl-(6S)-tetrahydrofolat je Tablette in % in Abhängigkeit vom angewandten Herstellungsprozess zeitlich unmittelbar nach der Herstellung. Der Unterschied zwischen Prozess 1 und Prozess 2 besteht im Zeitpunkt in dem das 5-Methyl-(6S)-tetrahydrofolat bei der Herstellung der untersuchten Tablette zugegeben wurde. In Prozess 1 war das 5-Methyl-(6S)-tetrahydrofolat bereits bei der Granulation in der Mischung vorhanden, während es in Prozess 2 erst im Anschluss an die Granulation aufgezogen wurde. Der Gehalt an 5-Methyl-(6S)-tetrahydrofolat in der nach Prozess 1 hergestellten Formulierung ist deutlich geringer.

**Tabelle 4: Metafolingehalt in Abhängigkeit vom Herstellungsprozess direkt nach der Herstellung**

| | Metafolingehalt Granulation (Prozess 1) | Metafolingehalt Aufziehen (Prozess 2) |
|---|---|---|
| Mittelwert | 88,5 | 96,1 % |
| Verteilungskoeffizient | 6,1 | 2,5 |

Tabelle 5 zeigt Gehalt an 5-Methyl-(6S)-tetrahydrofolat in Abhängigkeit vom verwendeten Herstellungsverfahren nach einmonatiger Lagerung bei bestimmten Temperaturen und Luftfeuchtigkeiten. Der in Tabelle 4 erkennbare Trend, dass bereits vor der Granulation zugesetztes 5-Methyl-(6S)-tetrahydrofolat instabiler ist, bestätigt sich insbesondere unter Lagerbedingungen von 40°C und 75 % relativer Luftfeuchtigkeit (rH).

**Tabelle 5: Metafolingehalt in Abhängigkeit vom Herstellungsprozess nach Lagerung**

| | 25°C / 60% rH Granulation (Prozess 1) | 25°C / 60% rH Aufziehen (Prozess 2) | 40°C / 75% rH Granulation (Prozess 1) | 40°C / 75% rH Aufziehen (Prozess 2) |
|---|---|---|---|---|
| Vials offen | 63,2 % | 92,1 % | 43,4 % | 67,7 % |
| Vials geschlossen | 74,5 % | 92,5 % | 58,9 % | 90,1 % |

Es ist bekannt, dass bei einer trockenen Zumischung die Freisetzung langsamer erfolgt als im Falle einer Granulation. Überraschenderweise wurde jedoch festgestellt, dass die trockene Zumischung des 5-Methyl-(6S)-tetrahydrofolats die Freisetzung nicht verzögert, sondern sogar beschleunigt. Hierzu wurden die Tabletten in einem In-vitro-Dissolution-Versuch mittels einer USP-paddle-Apparatur bei 50 rpm und 37°C in einer 0,03 prozentigen wässrigen Ascorbinsäurelösung untersucht. Tabelle 6 zeigt die Ergebnisse der In-vitro-Dissolution-Versuche.

**Tabelle 6: Dissolution in %**

| Zeit [min] | 5-Methyl-(6S)-tetrahydrofolat Prozess 1 Dissolution [%] | 5-Methyl-(6S)-tetrahydrofolat Prozess 2 Dissolution [%] |
|---|---|---|
| 0 | 0 | 0 |
| 10 | 59,2 | 81,4 |
| 15 | 66,8 | 89,3 |
| 30 | 73,1 | 91,3 |
| 45 | 76,7 | 91,1 |
| 60 | 75,8 | 91,2 |

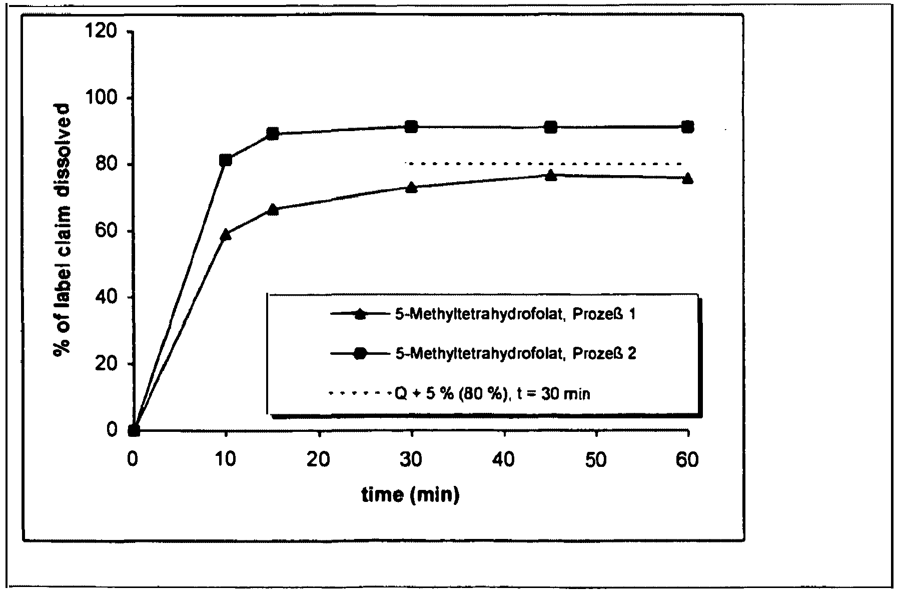

Die regelmäßige Einnahme der erfindungsgemäß hergestellten pharmazeutischen Zusammensetzung mit der besonders bevorzugten Dosis von 451 µg des Kalziumsalzes der 5-Methyl-(6S)-tetrahydrofolsäure pro Tag führt zu einem Anstieg der Folatkonzentrationen im Serum und Erythrozyten bis zum Erreichen eines Fließgleichgewichtes (steady state). Die entsprechende Erythrozytenfolat-Invasionskinetik wird durch eine Halbwertzeit von 6 bis 10 Wochen beschrieben. Auf Basis dieser Halbwertzeit ist das Erreichen von etwa 97% des Erythrozytenfolat steady state Spiegels nach ungefähr 5 Halbwertzeiten (entsprechend etwa 30 bis 50 Wochen) zu erwarten. Bei fortgesetzter, täglicher Einnahme der erfindungsgemäß hergestellten pharmazeutischen Zusammensetzung bleiben die Erythrozytenfolatspiegel im Bereich der steady state Konzentrationen. Nach Absetzen der erfindungsgemäß hergestellten pharmazeutischen Zusammensetzung sinken die Erythrozy-tenfolatspiegel langsam mit einer Halbwertzeit von ebenfalls etwa 6 bis 10 Wochen. Dadurch bleiben die Erythrozyten-folatspiegel auch ohne weitere, fortgesetzte Einnahme der erfindungsgemäße hergestellten pharmazeutischen Zusammensetzung mehrere Wochen in einem Bereich oberhalb der Grenze von 906 nmol/l, die allgemein als ausreichend zur Vermeidung von Neuralrohrdefekten angesehen wird. Das erfindungsgemäß hergestellte Präparat gewährleistet deshalb die Verminderung des Risikos folatmangelbedingter Erkrankungen und folatmangelbedingter angeborener Fehlbildungen, auch nach Beendigung einer längerfristigen Einnahme des Arzneimittels ("Pille").

Offenbart ist auch die Verwendung von 5-Methyl-(6S)-tetrahydrofolat, ein oder mehrerer Estrogene und Gestagene, sowie optional von Vitamin B₆ und/oder Vitamin B₂, sowie pharmazeutisch verträglicher Hilfs- und Trägerstoffe, in Abwesenheit von Vitamin B12, zur Herstellung eines Arzneimittels zur mindestens 8 Wochen nach der Beendigung einer vorangegangenen längerfristigen und fortgesetzten Einnahme dieses Arzneimittels andauernden Verminderung des Risikos von folatmangelbedingten Erkrankungen und folatmangelbedingten angeborenen Fehlbildungen.

Offenbart ist ebenfalls ein Kit enthaltend mindestens 20 tägliche Dosiseinheiten enthaltend das erfindungsgemäß hergestellte Arzneimittel und mindestens eine tägliche Dosiseinheit enthaltend das Kalziumsalz der 5-Methyl-(6S)-tetrahydrofolsäure, sowie optional Vitamin B6 und/oder Vitamin B₂, wobei die Anzahl aller im Kit enthaltenen Dosiseinheiten mindestens 28 beträgt und die Dosiseinheiten so angeordnet sind, dass zuerst die das erfindungsgemäß hergestellte Arzneimittel enthaltenden Dosiseinheiten und anschließend die weder Estrogen noch Gestagen enthaltenden Dosiseinheiten einzunehmen sind. Im Falle der zuerst genannten, das erfindungsgemäß hergestellte Arzneimittel enthaltenden, mindestens 20 täglichen Dosiseinheiten ist es auch möglich, das Kalziumsalz der 5-Methyl-(6S)-tetrahydrofolsäure getrennt zu formulieren und als zusätzliche Dosiseinheiten räumlich so anzuordnen, dass sich aus dieser Anordnung die gemeinsame Einnahme beider Dosiseinheiten ergibt.

Weitere Ausgestaltungen für verschiedene Kits sind ein Kit wie im vorhergehenden Absatz offenbart:
- enthaltend 20-30 tägliche Dosiseinheiten enthaltend ein hierin offenbartes Arzneimittel und 1 - 10 tägliche Dosiseinheiten enthaltend 5-Methyl-(6S)-tetrahydrofolat; oder
- enthaltend 21 - 26 tägliche Dosiseinheiten enthaltend ein hierin offenbartes Arzneimittel und 2 - 7 tägliche Dosiseinheiten enthaltend 5-Methyl-(6S)-tetrahydrofolat, wobei die Anzahl aller im Kit enthaltenen Dosiseinheiten 28 beträgt; oder
- enthaltend 21 tägliche Dosiseinheiten enthaltend ein hierin offenbartes Arzneimittel und 7 tägliche Dosiseinheiten enthaltend 5-Methyl-(6S)-tetrahydrofolat; oder
- enthaltend 24 tägliche Dosiseinheiten enthaltend ein hierin offenbartes Arzneimittel und 4 tägliche Dosiseinheiten enthaltend 5-Methyl-(6S)-tetrahydrofolat; oder
- enthaltend 451 µg des Kalziumsalzes der 5-Methyl-(6S)-tetrahydrofolsäure in jeder täglichen Dosiseinheit; oder
- enthaltend 21 tägliche Dosiseinheiten enthaltend ein wie in Anspruch 12 definiertes Arzneimittel und 7 tägliche Dosiseinheiten enthaltend 5-Methyl-(6S)-tetrahydrofolat; oder
- enthaltend 24 tägliche Dosiseinheiten enthaltend ein wie in Anspruch 12 definiertes Arzneimittel und 4 tägliche Dosiseinheiten enthaltend 5-Methyl-(6S)-tetrahydrofolat; oder
- enthaltend 21 tägliche Dosiseinheiten enthaltend ein wie in Anspruch 13 definiertes Arzneimittel und 7 tägliche Dosiseinheiten enthaltend 5-Methyl-(6S)-tetrahydrofolat.

Insbesondere ist es auch möglich, das erfindungsgemäß hergestellte Arzneimittel in einem so genannten "Extended Regime" zu verabreichen. Hierunter wird eine durchgängige, längere als 28 tägige Gabe des Arzneimittels verstanden, wobei der verlängerte Anwendungszyklus durch eine 1 bis 7 tägige Gabe ausschließlich 5-Methyl-(6S)-tetrahydrofolat haltiger Dosiseinheiten oder durch Einnahme von 1 bis 7 Placebos (Dosiseinheiten aktiven Wirkstoff) oder 1 bis 7 Blindpillentage (keine Verabreichung jedweder Dosiseinheit) vervollständigt wird.

Die nachfolgenden Beispiele dienen der näheren Erläuterung des Erfindungsgegenstandes, ohne ihn auf diese beschränken zu wollen.

### Beispiel 1:

Die Zusammensetzung erfindungsgemäß hergestellter Tabletten (80 mg) kann Tabelle 7 entnommen werden.

**Tabelle 7: Zusammensetzung erfindungsgemäße hergestellter Tabletten Inhaltsstoff Menge**

| Zusammensetzung | A | B | C | D |
|---|---|---|---|---|
| Drospirenon | 3 mg | 3 mg | --- | 3 mg |
| Ethinylestradiol* | 0,03 mg | 0,02 mg | --- | 0,03 mg |
| Metafolin | 0,451 mg | 0,451 mg | 0,451 mg | 0,451 mg |
| Vitamin B12 | --- | --- | --- | 0,1 mg |
| Lactose monohydrate | Bis 80 mg | bis 80 mg | bis 80 mg | bis 80 mg |
| Maisstärke | 16,40 mg | 16,40 mg | 16,40 mg | 16,40 mg |
| Maisstärke** | 2 mg*** | 2 mg*** | 2 mg*** | 2 mg*** |
| Modifizierte Maisstärke | 9,60 mg | 9,60 mg | 9,60 mg | 9,60 mg |
| Magnesium stearat | 0,80 mg | 0,80 mg | 0,80 mg | 0,80 mg |
| *: optional als Ethinylestradiol-beta-Cyclodextrin-Komplex; Die Mengenangabe bezieht sich dabei auf unkomplexiertes Ethinylestradiol. Im Falle der Verwendung des Ethinylestradiol-beta-Cyclodextrin-Komplex ist etwa die zehnfache Menge einzusetzen. Der Gehalt von Ethinylestradiol im β-Cyclodextrin-Komplex beträgt nämlich etwa 9,5 bis 12,5 % (vergleiche WO02/49675). | | | | |
| **: Der mit ** gekennzeichnete Teil der Maisstärke kann durch einen Alternativbinder, wie zum Beispiel 1,6 mg niedrig substituierter Hydroxypropylzellulose ersetzt werden. | | | | |
| ***: Die Menge der als Binder eingesetzten Maisstärke** kann zum Beispiel auch 1,8 mg betragen. | | | | |

Die Herstellung der oralen Formulierung erfolgt durch Mischen der oben genannten Bestandteile, Granulierung mit dem als Binder verwendetem Teil der Maisstärke, Aufziehen des Kalziumsalzes der 5-Methyl-(6S)-tetrahydrofolsäure nach Abschluss des Granulationsprozesses, erneutem Mischen, Tablettierung und Befilmung.

### Beispiel 2:

80 gesunden jungen Frauen im gebärfähigen Alter wird im Abstand von 8 Wochen Blut abgenommen und der Erythrozytenfolatspiegel mit einer validierten mikrobiologischen, immunologischen oder instrumentellen (z. B. HPLC, LC-MS/MS) Methode oder einer geeigneten Kombination dieser Methoden bestimmt.

8 Wochen nach der ersten Blutentnahme (Screeningphase) wird über einen Zeitraum von 40 Wochen:
täglich 451 µg des Kalziumsalzes von 5-Methyl-(6S)-tetrahydrofolsäure oder alternativ:
in den jeweils ersten 21 Tagen des jeweiligen Zyklus gleichzeitig 3 mg Drospirenon, 30 µg Ethinylestradiol und 451 µg des Kalziumsalzes der 5-Methyl-(6S)-tetrahydrofolsäure verabreicht (Tablette gemäß Zusammensetzung A nach Ausführungsbeispiel 1). In einer sich daran unmittelbar anschließenden Phase wird die Gabe von 451 µg des Kalziumsalzes der 5-Methyl-(6S)-tetrahydrofolsäure für 7 Tage fortgesetzt (Zusammensetzung C). Für weitere 21 Tage (zweiter Zyklus) werden erneut 3 mg Drospirenon, 30 µg Ethinylestradiol und 451 µg des Kalziumsalzes der 5-Methyl-(6S)-tetrahydrofolsäure (Zusammensetzung A) und für weitere 7 Tage nur 451 µg des Kalziumsalzes der 5-Methyl-(6S)-tetrahydrofolsäure (Zusammensetzung C) und so weiter (Medikationsphase) appliziert.

Nach 48 Wochen wird kein 5-Methyl-(6S)-tetrahydrofolat mehr verabreicht. Alternativ können Drospirenon und Ethinylestradiol für weitere 40 Wochen weiter verabreicht oder ebenfalls abgesetzt werden.

Die letzte Blutentnahme erfolgt nach 88 Wochen. Die drop out rate kann wegen des Langzeitcharakters der Studie bis zu 50 % betragen.

### Beispiel 3:

80 gesunden jungen Frauen im gebärfähigen Alter wird im Abstand von 8 Wochen Blut abgenommen und der Erythrozytenfolatspiegel mit einer validierten mikrobiologischen, immunologischen oder instrumentellen (z. B. HPLC, LC-MS/MS) Methode oder einer geeigneten Kombination dieser Methoden bestimmt.

8 Wochen nach der ersten Blutentnahme wird über einen Zeitraum von 40 Wochen in den jeweils ersten 24 Tagen des jeweiligen Zyklus gleichzeitig 3 mg Drospirenon, 20 µg Ethinylestradiol und 451 µg des Kalziumsalzes der 5-Methyl-(6S)-tetrahydrofolsäure verabreicht (Zusammensetzung B). In einer sich daran unmittelbar anschließenden Phase wird die Gabe von 451 µg des Kalziumsalzes der 5-Methyl-(6S)-tetrahydrofolsäure für 7 Tage fortgesetzt (Zusammensetzung C). Für weitere 21 Tage (zweiter Zyklus) werden erneut 3 mg Drospirenon, 20 µg Ethinylestradiol und 451 µg des Kalziumsalzes der 5-Methyl-(6S)-tetrahydrofolsäure (Zusammensetzung B) und für weitere 7 Tage nur 451 µg des Kalziumsalzes der 5-Methyl-(6S)-tetrahydrofolsäure (Zusammensetzung C) und so weiter appliziert.

Nach 48 Wochen wird kein 5-Methyl-(6S)-tetrahydrofolat mehr verabreicht, während Drospirenon und Ethinylestradiol für weitere 40 Wochen weiter verabreicht oder ebenfalls abgesetzt wird.

Die letzte Blutentnahme erfolgt nach 88 Wochen. Die drop out rate kann wegen des Langzeitcharakters der Studie bis zu 50 % betragen.

Der Ausgangswert des Erythrozytenfolatspiegels der Probanden liegt je nach Ernährungsgewohnheiten bei etwa 500 bis 700 nmol/l, in jedem Fall aber unter 906 nmol/l. Dieser Wert steigt bei gleichbleibenden Ernährungsgewohnheiten mit Applikation der erfindungsgemäßen pharmazeutischen Zusammensetzung in den folgenden Tagen an und erreicht bereits nach 6 bis 8 Wochen - also nach dem zweiten Zyklus - einen Wert von etwa 906 nmol/l. Nach andauernder Verabreichung von mindestens 30 Wochen (entsprechend dem fünffachen Wert der unteren Grenze der Halbwertszeit) wird bei gleichbleibenden Ernährungsgewohnheiten ein Erythrozytenfolatspiegel von ungefähr 1200 bis 1600 nmol/l erreicht (steady state). Nach Beendigung der Applikation des 5-Methyl-(6S)-tetrahydrofolats sinkt der Erythrozytenfolatspiegel kontinuierlich ab. Ausgehend von einer mittleren steady state Konzentration von 1400 nmol/l erfolgt bei gleichbleibenden Ernährungsgewohnheiten die Unterschreitung eines Erythrozytenfolatspiegels von 906 nmol/l und damit der allgemein zur Vermeidung von Neuralrohrdefekten ausreichenden Mindestkonzentration in Erythrozyten voraussichtlich in der elften bis dreizehnten Woche nach dem Absetzen der erfindungsgemäßen pharmazeutischen Zusammensetzung.

### Beispiel 4: Langzeit - Folat Studie

180 gesunden jungen Frauen im gebärfähigen Alter (zur Hälfte davon erhalten mit Folsäure angereicherte Nahrung) wird im Abstand von 2 Wochen Blut abgenommen und der Erythrozytenfolatspiegel mit einer validierten mikrobiologischen, immunologischen oder instrumentalen (z. B. HPLC, LC-MS/MS) Methode oder einer geeigneten Kombination dieser Methoden bestimmt.

8 Wochen nach der ersten Blutabnahme wird einer ersten Gruppe von 90 Frauen über einen Zeitraum von 24 Wochen
in den jeweils ersten 21 Tagen des jeweiligen Zyklus gleichzeitig 3 mg Drospirenon, 30 µg Ethinylestradiol und 451 µg des Kalziumsalzes der 5-Methyl-(6S)-tetrahydrofolsäure verabreicht. In einer sich daran unmittelbar anschließenden Phase wird die Gabe von 451 µg des Kalziumsalzes der 5-Methyl-(6S)-tetrahydrofolsäure für 7 Tage fortgesetzt. Für weitere 21 Tage (zweiter Zyklus) werden erneut 3 mg Drospirenon, 30 µg Ethinylestradiol und 451 µg des Kalziumsalzes der 5-Methyl-(6S)-tetrahydrofolsäure und für weitere 7 Tage nur 451 µg des Kalziumsalzes der 5-Methyl-(6S)-tetrahydrofolsäure und so weiter (Medikationsphase) appliziert.

Als Kontrollgruppe werden einer Gruppe von 90 Frauen nach dem gleichen Verabreichungsschema 3 mg Drospirenon, 30 µg Ethinylestradiol und 400 µg Folsäure verabreicht.

Die letzte Blutentnahme erfolgt in beiden Fällen nach 24 Wochen. Daran schließt sich eine Nachbeobachtungszeit von 20 Wochen an, in der 20 Wochen lang das empfängnisverhütende Präparat Yasmin^{®} verabreicht wird, d.h. in den jeweils ersten 21 Tagen des jeweiligen Zyklus werden gleichzeitig 3 mg Drospirenon und 30 µg Ethinylestradiol verabreicht; unmittelbar daran anschließend wird 7 Tage lang kein Wirkstoff verabreicht (Placebos oder keine Verabreichung). Die drop out rate kann bis zu 30 % betragen.

Der Ausgangswert des Erythrozytenfolatspiegels der Probanden liegt unter 906 nmol/l. Dieser Wert steigt bei gleich bleibenden Ernährungsgewohnheiten mit Applikation der erfindungsgemäßen pharmazeutischen Zusammensetzung in den folgenden Tagen an und erreicht bei der Mehrheit der Frauen bereits nach 6 bis 8 Wochen einen Wert von etwa 906 nmol/l. Nach andauernder Verabreichung von 24 Wochenwird bei gleich bleibenden Ernährungsgewohnheiten in beiden Gruppen ein Erythrozytenfolatspiegel erreicht, der die Equivalenz zwischen beiden Behandlungsgruppen zeigt (Bioequivalenzkriterium 80 - 125 %). Nach Beendigung der Applikation des 5-Methyl-(6S)-tetrahydrofolats sinkt der Erythrozytenfolatspiegel kontinuierlich ab. Es wird ermittelt, wann der Erythrozytenfolatspiegel die anerkannte Schwelle von 906 nmol/l, die allgemein zur Vermeidung von Neuralrohrdefekten als ausreichend angesehen wird, unterschreitet.

Die Mehrzahl der Frauen in der ersten Gruppe weist noch 3 Monate nach Beendigung der Einnahme einen solchen ausreichenden Erythrozytenfolatspiegel auf.

## Patentansprüche

1. Verfahren zur Formulierung eines Arzneimittels enthaltend
- eine tägliche Dosis von 0,4 bis 1 mg des Kalziumsalzes der 5-Methyl-(6S)-tetrahydrofolsäure,
- ein oder mehrere Estrogene und Gestagene,
- optional Vitamin B6 und/oder Vitamin B2,
- sowie pharmazeutisch verträgliche Hilfs-/Trägerstoffe
in der Abwesenheit von Vitamin B12,
**dadurch gekennzeichnet, dass** das Kalziumsalz der 5-Methyl-(6S)-tetrahydrofolsäure erst nach dem Granulieren aufgezogen wird.

2. Verfahren zur Formulierung eines Arzneimittels nach Anspruch 1 enthaltend mindestens ein Estrogen, ausgewählt aus der Gruppe von Ethinylestradiol, Mestranol, Quinestranol, Estradiol, Estron, Estran, Estriol, Estetrol und konjugierte equine Estrogene.

3. Verfahren zur Formulierung eines Arzneimittels nach Anspruch 1 enthaltend mindestens ein Gestagen, ausgewählt aus der Gruppe von Levonorgestrel, Norgestimat, Norethisteron, Dydrogesteron, Drospirenon, 3-beta-Hydroxydesogestrel, 3-Ketodesogestrel (=Etonogestrel), 17-Deacetylnorgestimat, 19-Norprogesteron, Acetoxypregnenolon, Allylestrenol, Amgeston, Chlormadinon, Cyproteron, Demegeston, Desogestrel, Dienogest, Dihydrogesteron, Dimethisteron, Ethisteron, Ethynodioldiacetat, Flurogestonacetat, Gastrinon, Gestoden, Gestrinon, Hydroxymethylprogesteron, Hydroxyprogesteron, Lynestrenol (=Lynoestrenol), Mecirogeston, Medroxyprogesteron, Megestrol, Melengestrol, Nomegestrol, Norethindron (= Norethisteron), Norethynodrel, Norgestrel (einschließlich d- Norgestrel und dl-Norgestrel), Norgestrienon, Normethisteron, Progesteron, Quingestanol, (17alpha)-17-Hydroxy-11-methylen-19-norpregna-4,15-dien-20-yn-3-on, Tibolon, Trimegeston, Algestonacetophenid, Nestoron, Promegeston, 17-Hydroxyprogesteronester, 19-Nor-17hydroxyprogesteron, 17alpha-Ethinyl-testosteron, 17alpha-ethinyl-19-nor-testosteron, d-17beta-Acetoxy-13beta-ethyl-17alpha-ethinyl-gon-4-en-3-onoxim oder Tanaproget.

4. Verfahren zur Formulierung eines Arzneimittels nach Anspruch 1 enthaltend ein kristallines Kalziumsalz der 5-Methyl-(6S)-tetrahydrofolsäure.

5. Verfahren zur Formulierung eines Arzneimittels nach Anspruch 1 enthaltend das Kalziumsalz des 5-Methyl-(6S)-tetrahydrofolsäure, Drospirenon und Ethinylestradiol.

6. Verfahren zur Formulierung eines Arzneimittels nach Anspruch 5 enthaltend eine tägliche Dosis von 451 µg des Kalziumsalzes der 5-Methyl-(6S)-tetrahydrofolsäure.

7. Verfahren zur Formulierung eines Arzneimittels nach Anspruch 5 enthaltend eine tägliche Dosis von 3 mg Drospirenon.

8. Verfahren zur Formulierung eines Arzneimittels nach Anspruch 5 enthaltend eine tägliche Dosis von 10 bis 50 µg Ethinylestradiol.

9. Verfahren zur Formulierung eines Arzneimittels nach Anspruch 5 enthaltend eine tägliche Dosis von 10 bis 30 µg Ethinylestradiol.

10. Verfahren zur Formulierung eines Arzneimittels nach Anspruch 5 enthaltend eine tägliche Dosis von 20 µg Ethinylestradiol.

11. Verfahren zur Formulierung eines Arzneimittels nach Anspruch 5 enthaltend eine tägliche Dosis von 30 µg Ethinylestradiol.

12. Verfahren zur Formulierung eines Arzneimittels nach Anspruch 5 enthaltend
- eine tägliche Dosis von 451 µg des Kalziumsalzes der 5-Methyl-(6S)- tetrahydrofolsäure,
- eine tägliche Dosis von 3 mg Drospirenon und
- eine tägliche Dosis von 20 µg Ethinylestradiol.

13. Verfahren zur Formulierung eines Arzneimittels nach Anspruch 5 enthaltend
- eine tägliche Dosis von 451 µg des Kalziumsalzes der 5-Methyl-(6S)- tetrahydrofolsäure,
- eine tägliche Dosis von 3 mg Drospirenon und
- eine tägliche Dosis von 30 µg Ethinylestradiol.

## Claims

1. Method for formulating a medicament comprising
- a daily dose of 0.4 to 1 mg of the calcium salt of 5-methyl-(6S)-tetrahydrofolic acid
- one or more estrogens and progestogens,
- optionally vitamin B₆ and/or vitamin B₂,
- and pharmaceutically acceptable excipients/ carriers
in the absence of vitamin B₁₂,
**characterized in that** the calcium salt of 5-methyl-(6S)-tetrahydrofolic acid is absorbed only after the granulation.

2. Method for formulating a medicament according to Claim 1 comprising at least one estrogen selected from the group of ethinylestradiol, mestranol, quinestranol, estradiol, estrone, estrane, estriol, estetrol and conjugated equine estrogens.

3. Method for formulating a medicament according to Claim 1 comprising at least one progestogen selected from the group of levonorgestrel, norgestimate, norethisterone, dydrogesterone, drospirenone, 3-beta-hydroxydesogestrel, 3-ketodesogestrel (= etonogestrel), 17-deacetylnor-gestimate, 19-norprogesterone, acetoxypregneno-lone, allylestrenol, amgestone, chlormadinone, cyproterone, demegestone, desogestrel, dienogest, dihydrogesterone, dimethisterone, ethisterone, ethynodiol diacetate, flurogestone acetate, gastrinone, gestodene, gestrinone, hydroxymethyl-progesterone, hydroxyprogesterone, lynestrenol (= lynoestrenol), mecirogestone, medroxyproge-sterone, megestrol, melengestrol, nomegestrol, norethindrone (= norethisterone), norethynodrel, norgestrel (including d-norgestrel and dl-norgestrel), norgestrienone, normethisterone, progesterone, quingestanol, (17alpha)-17-hydroxy-11-methylene-19-norpregna-4,15-dien-20-yn-3-one, tibolone, trimegestone, algestoneacetophenide, nestorone, promegestone, 17-hydroxyprogesterone esters, 19-nor-17hydroxyprogesterone, 17alpha-ethynyltestosterone, 17alpha-ethynyl-19-norte-stosterone, d-17beta-acetoxy-13beta-ethyl-17alpha-ethynylgon-4-en-3-one oxime or tanaproget.

4. Method for formulating a medicament according to Claim 1 comprising a crystalline calcium salt of 5-methyl-(6S)-tetrahydrofolic acid.

5. Method for formulating a medicament according to Claim 1 comprising the calcium salt of 5-methyl-(6S)-tetrahydrofolic acid, drospirenone and ethinylestradiol.

6. Method for formulating a medicament according to Claim 5 comprising a daily dose of 451 µg of the calcium salt of 5-methyl-(6S)-tetrahydrofolic acid.

7. Method for formulating a medicament according to Claim 5 comprising a daily dose of 3 mg of drospirenone.

8. Method for formulating a medicament according to Claim 5 comprising a daily dose of 10 to 50 µg of ethinylestradiol.

9. Method for formulating a medicament according to Claim 5 comprising a daily dose of 10 to 30 µg of ethinylestradiol.

10. Method for formulating a medicament according to Claim 5 comprising a daily dose of 20 µg of ethinylestradiol.

11. Method for formulating a medicament according to Claim 5 comprising a daily dose of 30 µg of ethinylestradiol.

12. Method for formulating a medicament according to Claim 5 comprising
- a daily dose of 451 µg of the calcium salt of 5-methyl-(6S)-tetrahydrofolic acid,
- a daily dose of 3 mg of drospirenone and
- a daily dose of 20 µg of ethinylestradiol.

13. Method for formulating a medicament according to Claim 5 comprising
- a daily dose of 451 µg of the calcium salt of 5-methyl-(6S)-tetrahydrofolic acid,
- a daily dose of 3 mg of drospirenone and
- a daily dose of 30 µg of ethinylestradiol.

## Revendications

1. Procédé de formulation d'un médicament contenant
- une dose journalière de 0,4 à 0,1 mg du sel de potassium de l'acide 5-méthyl-(6S)-tétrahydrofolique,
- un ou plusieurs estrogènes et gestagènes,
- éventuellement de la vitamine B₆ et/ou de la vitamine B₂,
- ainsi que des substances auxiliaires/de support pharmaceutiquement acceptables
en l'absence de vitamine B₁₂,
**caractérisé en ce que** le sel de potassium de l'acide 5-méthyl-(6S)-tétrahydrofolique n'est appliqué qu'après la granulation.

2. Procédé de formulation d'un médicament selon la revendication 1 contenant au moins un estrogène, choisi dans le groupe formé par l'éthynylestradiol, le mestranol, le quinestranol, l'estradiol, l'estron, l'estran, l'estriol, l'estétrol et des estrogènes équins conjugués.

3. Procédé de formulation d'un médicament selon la revendication 1 contenant au moins un gestagène choisi dans le groupe formé par le lévonorgestrel, le norgestimate, la noréthistérone, la dydrogestérone, la drospirénone, le 3-bêta-hydroxydésogestrel, le 3-cétodésogestrel (= étonogestrel), le 17-désacétylnorgestimate, la 19-norprogestérone, l'acétoxypregnénolone, l'allylestrénol, l'amgeston, la chlormadinone, la cyprotérone, la démegestone, le désogestrel, le diénogest, la dihydrogestérone, la diméthistérone, l'éthistérone, le diacétate d'éthynodiol, l'acétate de fluorgestone, la gastrinone, le gestodène, la gestrinone, l'hydroxyméthylprogestérone, l'hydroxyprogestérone, le lynestrénol (= Lynoestrénol), la mécirogestone, la médroxyprogestérone, le mégestrol, le mélengestrol, le nomégestrol, la noréthindrone (= noréthistérone), le noréthynodrel, le norgestrel (y compris le d-norgestrel et le dl-norgestrel), la norgestriénone, la norméthistérone, la progestérone, le quingestanol, la (17alpha)-17-hydroxy-11-méthylène-19-norpregna-4,15-dién-20-yn-3-one, la tibolone, la trimégestone, l'algestonacétophénide, la nestorone, la promégestone, le 17-hydroxyprogestéronester, la 19-nor-17-hydroxyprogestérone, la 17-alpha-éthynyl-testostérone, la 17-alpha-éthynyl-19-nortestostérone, la d-17-bêta-acétoxy-13-bêta-éthyl-17-alpha-éthynyl-gon-4-én-3-onoxime ou le tanaproget.

4. Procédé de formulation d'un médicament selon la revendication 1 contenant un sel cristallin de potassium de l'acide 5-méthyl-(6S)-tétrahydrofolique.

5. Procédé de formulation d'un médicament selon la revendication 1 contenant le sel de potassium de l'acide 5-méthyl-(6S)-tétrahydrofolique, de la drospirénone et de l'éthynylestradiol.

6. Procédé de formulation d'un médicament selon la revendication 5 contenant une dose journalière de 451 µg du sel de potassium de l'acide 5-méthyl-(6S)-tétrahydrofolique.

7. Procédé de formulation d'un médicament selon la revendication 5 contenant une dose journalière de 3 mg de drospirénone.

8. Procédé de formulation d'un médicament selon la revendication 5 contenant une dose journalière de 10 à 50 µg d'éthynylestradiol.

9. Procédé de formulation d'un médicament selon la revendication 5 contenant une dose journalière de 10 à 30 µg d'éthynylestradiol.

10. Procédé de formulation d'un médicament selon la revendication 5 contenant une dose journalière de 20 µg d'éthynylestradiol.

11. Procédé de formulation d'un médicament selon la revendication 5 contenant une dose journalière de 30 µg d'éthynylestradiol.

12. Procédé de formulation d'un médicament selon la revendication 5 contenant
- une dose journalière de 451 µg du sel de potassium de l'acide 5-méthyl-(6S)-tétrahydrofolique,
- une dose journalière de 3 mg de drospirénone et
- une dose journalière de 20 µg d'éthynylestradiol.

13. Procédé de formulation d'un médicament selon la revendication 5 contenant
- une dose journalière de 451 µg du sel de potassium de l'acide 5-méthyl-(6S)-tétrahydrofolique,
- une dose journalière de 3 mg de drospirénone et
- une dose journalière de 30 µg d'éthynylestradiol.
